# EUROPÄISCHE PATENTSCHRIFT

(11) **EP 3 246 033 B1**
(45) Veröffentlichungstag und Bekanntmachung des Hinweises auf die Patenterteilung: **30.09.2020**
(21) Anmeldenummer: 17165209.2
(22) Anmeldetag: 06.04.2017
(51) Int. Cl.: A61K 31/65, A61K 31/661, A61K 31/7036, A61K 31/717, A61K 31/728, A61K 31/795, A61K 45/06, A61P 19/02, A61L 2/00

(54) **POLYMERLÖSUNG ZUR VISCOSUPPLEMENTATION ENTHALTEND POLYSACCHARID UND POLYSTYRENSULFONSÄURE**
POLYMER SOLUTION FOR VISCOSUPPLEMENTATION COMPRISING POLYSACCHARIDE AND POLYSTYRENE SULFONIC ACID
SOLUTION POLYMÈRE POUR VISCOSUPPLÉMENTATION CONTENANT DES POLYSACCHARIDES ET DE L'ACIDE POLYSTYRÈNE SULFONIQUE

(30) Priorität: 19.05.2016 DE 102016208567
(43) Veröffentlichungstag der Anmeldung: 22.11.2017
(73) Patentinhaber: Heraeus Medical GmbH, 61273 Wehrheim (DE)
(72) Erfinder: Kluge, Thomas, 56179 Vallendar (DE); Vogt, Sebastian, 99092 Erfurt (DE)
(74) Vertreter: Heraeus IP

(56) Entgegenhaltungen:
- EP-A1- 1 031 345
- EP-A1- 3 184 550
- EP-B1- 2 596 812
- WO-A1-2009/103123
- DE-A1- 10 032 118
- US-A- 4 782 046
- T. ISOYAMA: "Differential selectivity of hyaluronidase inhibitors toward acidic and basic hyaluronidases", GLYCOBIOLOGY, Bd. 16, Nr. 1, 13. Juli 2005 (2005-07-13), Seiten 11-21, XP055406634, US ISSN: 0959-6658, DOI: 10.1093/glycob/cwj036

## Beschreibung

Gegenstand der Erfindung ist eine Polymerlösung zur Verwendung bei einer therapeutischen Viscosupplementation, insbesondere zur Behandlung von Arthrose.

Arthrose (*Arthrosis deformans*) ist eine weit verbreitete degenerative Erkrankung der Gelenke. Dabei kommt es zu einer Schädigung (Erosion) der Knorpeloberflächen, der Ablösung von Knorpelpartikeln und durch Knorpelpartikel verursachte Entzündungen der Synovialmembran. Bei leichter und mittelschwerer Arthrose wird seit einigen Jahren versucht, durch eine intraartikuläre Injektion von Hyaluronsäure (Viscosupplementation) die Schmerzsituation der Patienten zu verbessern und gleichzeitig das Fortschreiten der Arthrose zu vermindern.

Hyaluronsäure ist ein natürlicher Bestandteil der Gelenkflüssigkeit (Synovialflüssigkeit). Die Hyaluronsäure wirkt in der Gelenkflüssigkeit als Schmiermittel. Besonders vorteilhaft ist es, dass wässrige Hyaluronsäurelösungen Viscoelastisch sind. Dadurch ergeben sich sehr gute Schmier- und Gleiteigenschaften.

Auf Grund der vorteilhaften Schmiereigenschaften werden wässrige Hyaluronsäurelösungen seit ungefähr zwei Jahrzehnten zur Viscosupplementation verwendet. Stand der Technik ist gegenwärtig die Verwendung von fermentativ hergestellter Hyaluronsäure. Daneben erscheint auch die Verwendung von in Wasser löslichen Cellulosederivaten, wie Carboxymethylcellulose und Methylcellulose, von Stärkederivaten, wie Hydroxyethylstärke, zur Viscosupplementation grundsätzlich möglich.

Für die Viscosupplementation wird üblicherweise eine sterile, wässrige Lösung der Hyaluronsäure verwendet. Problematisch ist bei der Verwendung von wässrigen Lösungen von Hyaluronsäuren, dass diese nach recht kurzer Zeit enzymatisch nach der Einspritzung in den Gelenkspalt durch körpereigene Hyaluronidasen abgebaut werden und dadurch die gewünschte Schmiermittelwirkung nachläßt. Wünschenswert sind daher wässrige Polymerlösungen, die stabiler gegenüber den körpereigenen Hyaluronidasen sind.

Die WO 2009/103123 A1 offenbart die Verwendung von acrylsäurehaltigen, insbesondere anionischen Dendrimerverbindungen mit Oberflächengruppen als Inhibitoren von Hyaluronidase und ihre Verwendung zur Behandlung von Krankheiten.

Die Aufgabe der Erfindung besteht in der Entwicklung einer wässrigen Polymerlösung, die in Wasser zu mindestens teilweise lösliche Polymere enthält, welche durch Hyaluronidasen nicht abgebaut werden kann. Die zu entwickelnde Polymerlösung soll stabiler als wässrige Hyaluronsäurelösungen gegenüber Hyaluronidase sein.

Eine weitere Aufgabe der Erfindung ist es, Kombinationen von in Wasser zu mindestens teilweise löslichen Polymeren zu finden, die in wässriger Lösung miteinander verträglich sind. Das bedeutet, diese Polymerkombinationen dürfen nicht zur Ausflockung eines oder beider Polymere in wässriger Lösung führen. Die Polymerkombinationen müssen sichtklare, wässrige Polymerlösungen bilden.

Eine weitere Aufgabe der Erfindung ist es, ein Verfahren zur Sterilisation der zu entwickelnden Polymerlösung zu entwickeln. Dieses Verfahren soll eine Sterilisation ohne Verfärbungen der Polymerlösungen ermöglichen. Das Sterilisationsverfahren muss gewährleisten, dass die beiden zu mindestens teilweise in Wasser löslichen Polymere nicht durch den Sterilisationsprozess aus der Polymerlösung ausflocken und der Lösungszustand der Polymere erhalten bleibt.

Die Aufgabe der Erfindung wird gemäß Anspruch 1 gelöst.

Erfindungsgemäß ist eine Polymerlösung zur Verwendung bei der therapeutischen Viscosupplementation, insbesondere für die Behandlung von Arthrose. Diese Polymerlösung enthält
a) mindestens ein zu mindestens teilweise in Wasser lösliches Polysaccharid, das aus der Gruppe ausgewählt ist, die aus Alkalisalzen der Hyaluronsäure, Alkalisalzen der Carboxymethylcellulose, Methylcellulose und Hydroxyethylcellulose besteht,
b) mindestens ein in Wasser lösliches Alkalisalz oder Erdalkalisalz der Polystyrensulfonsäure und
c) Wasser
   und wobei die Lösung sichtklar ist.

Überraschend wurde gefunden, dass wässrige Polymerlösungen, die in Wasser lösliche Polysaccharide, wie Hyaluronsäure, und in Wasser lösliche Polysacchariderivate, wie Carboxymethylcellulose, Methylcellulose und Hydroxyethylcellulose, und Alkali- und Erdalkalisalze von Polystyrolsulfonsäuren enthalten, nicht ausflockende, sichtklare Lösungen bilden. Weiterhin wurde gefunden, dass wässrige Lösungen, die Hyaluronsäure und Alkali- oder Erdalkalisalze der Polystyrolsulfonsäure enthalten, stabil gegenüber einer Degradation durch Hyaluronidasen sind.

Erfindungsgemäß ist auch eine Polymerlösung zur Verwendung bei der therapeutischen Viscosupplementation die aus
a) mindestens einem zu mindestens teilweise in Wasser löslichen Polysaccharid, das aus der Gruppe ausgewählt ist, die aus Alkalisalzen der Hyaluronsäure, Alkalisalzen der Carboxymethylcellulose, Methylcellulose und Hydroxyethylcellulose besteht,
b) mindestens einem in Wasser lösliches Alkalisalz oder Erdalkalisalz der Polystyrensulfonsäure,
c) Wasser,
d) optional 3- Hydroxypropionsäure,
e) optional mindestens einem Antiphlogistikum,
f) optional mindestens einem Antibiotikum,
g) optional mindestens einem Immunsuppressivum,
h) optional mindestens einem Cytostatikum besteht.

Es ist auch eine Polymerlösung zur Verwendung bei der therapeutischen Viscosupplementation erfindungsgemäß, die
a) mindestens ein zu mindestens teilweise in Wasser lösliches Polysaccharid, das aus der Gruppe ausgewählt ist, die aus Alkalisalzen der Hyaluronsäure, Alkalisalzen der Carboxymethylcellulose, Methylcellulose und Hydroxyethylcellulose besteht,
b) mindestens ein in Wasser lösliches Alkalisalz oder Erdalkalisalz der Polystyrensulfonsäure,
c) Wasser und
d) 3-Hydroxypropionsäure
   enthält.

Die Molmasse Mₙ der Polysaccharide liegt bevorzugt zwischen 20 000 und 3 000 000 Dalton. Besonders bevorzugt zwischen 50 000 und 2 000 000 Dalton.

Die Molmasse Mₙ des Hyaluronsäuresalzes liegt bevorzugt zwischen 100 000 und 2 000 000 Dalton. Besonders bevorzugt zwischen 500 000 und 1 500 000 Dalton.

Erfindungsgemäß handelt es sich bei dem Polystyrensulfonsäuresalz um das Salz eines Polymers mit den folgenden Struktureinheiten:

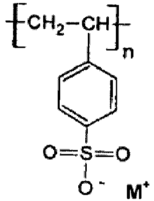

Dabei steht n für die Anzahl der sich wiederholenden Struktureinheiten und M⁺ steht für Alkali- und Erdalkaliionen. Polystyrensulfonsäuresalze haben bevorzugt eine Molmasse Mₙ von 20 000 bis 3 000 000 Dalton. Bevorzugt hat das Polystyrensulfonsäuresalz eine mittlere Molmasse Mₙ von 50 000 bis 2 000 000 Dalton. Als Alkali- und Erdalkalisalze der Polystyrensäure sind Natrium-, Kalium- und Magnesiumsalze bevorzugt. Besonders bevorzugt sind Natrium und Kaliumsalze.

Die Polymerlösung ist dadurch charakterisiert, dass das Massenverhältnis von Polysaccharid zu Alkalisalz oder Erdalkalisalz der Polystyrensulfonsäure 1,0 zu 1,0 bis 1 zu 0,0001 ist.

Bevorzugt beträgt der Gesamtpolymergehalt in der Polymerlösung von 0,1 bis 10 Gew.-%, besonders bevorzugt von 0,25 bis 5 Gew.-%.

Es ist vorteilhaft, wenn die Polymerlösung wenigstens ein Antiphlogistikum, wenigstens ein Antibiotikum, wenigstens ein Immunsuppressivum, wenigstens ein Cytostatikum oder eine Mischung aus diesen enthält.

Das wenigstens eine Antiphlogistikum ist vorzugsweise aus der Gruppe ausgewählt, die aus nicht-steroidalen Antiphlogistika und Glukokortikoiden besteht. Beispiele hierfür sind Acetylsalicylsäure, Ibuprofen, Diclofenac, Ketoprofen, Dexamethasonphosphat, Triamcinolon, Prednison, Hydrocortison, Hydrocortisonacetat und Fluticason. Besonders bevorzugt ist das wenigstens eine Antiphlogistikum aus der Gruppe ausgewählt, die aus Dexamethasonphosphat und Triamcinolon besteht.

Die Polymerlösung kann übliche Antibiotika, wie Gentamicinsulfat enthalten. Beispiele sind ferner Aminoglykosid-Antibiotika und Lincosamid-Antibiotika. Bevorzugt ist das mindestens eine Antibiotikum ist jedoch aus der Gruppe ausgewählt, die aus Tetracyclin-Antibiotika besteht, besonders bevorzugt ist es aus der Gruppe ausgewählt, die aus Doxycyclin, Chlortetracyclin und Oxytetracyclin besteht.

Ferner können Immunsuppressiva und Cytostatika enthalten sein. Besonders bevorzuge Beispiele sind Doxorubicin, Ciclosporin, Methotrexat, Leflunomid, Azathioprin, Mitomycin C, Tacrolimus, Sirolimus und Everolimus.

Besonders bevorzugt enthält die Polymerlösung mindestens einen Wirkstoff, der aus der Gruppe ausgewählt ist, die aus Dexamethasonphosphat, Triamcinolon, Doxycyclin, Chlortetracyclin, Oxytetracyclin, Doxorubicin, Ciclosporin, Methotrexat, Leflunomid, Azathioprin, Mitomycin C, Tacrolimus, Sirolimus und Everolimus besteht.

Die Antipholgistika, insbesondere Dexamethasonphosphat und Triamcinolon, können bei Verwendung der wässrigen Polymerlösung zur Viscosupplementation die Entzündungsprozesse am geschädigten Knorpelgewebe positiv beeinflussen. Weiterhin können Immunsuppressiva wie Mitomycin C, Tacrolimus, Sirolimus und Everolimus vorteilhaft der Polymerlösung zugesetzt werden. Erfindungsgemäß können auch Antibiotika, wie Doxycyclin, Chlortetracyclin, Oxytetracyclin der Polymerlösung zugesetzt werden.

Die bisher zur Viscosupplementation verwendeten Hyaluronsäurelösungen werden in erster Linie durch Gammabestrahlung sterilisiert. Dabei sind Dosen von gleich/größer 25 kGy üblich. Die Sterilisation wird an endverpackter Hyaluronsäurelösung vorgenommen. Die Gammabestrahlung ist jedoch mit gravierenden Nachteilen verbunden. Durch die Einwirkung von Gammastrahlung werden die Polymerketten abgebaut, wodurch sich die Molmasse deutlich verringert und niedermolekulare Zerfallsprodukte entstehen. Außerdem können die Packmittel, meistens Einwegspritzen aus Kunststoff, durch Gammastrahlung verspröden. Infolge von Nebenreaktionen treten ferner Verfärbungen der Hyaluronsäurelösungen auf. Insbesondere der Polymerabbau hängt wesentlich von der Dosis der Gammastrahlung ab. Übliche Gammaquellen haben ein kugelförmiges Strahlenfeld. Das bedeutet, dass die einwirkende Dosis in Abhängigkeit von der Position des zu sterilisierenden Gegenstandes variieren kann. Dadurch erfolgt der Polymerabbau nie gleichmäßig und Inhomogenitäten hinsichtlich der finalen Viskosität sind möglich. Eine reproduzierbare Endviskosität der sterilisierten Hyaluronsäurelösungen ist schwierig zu erreichen.

Ein alternatives Sterilisationsverfahren ist die Dampfsterilisation von wässrigen Hyaluronsäurelösungen, die jedoch zu Schädigungen an der Hyaluronsäure und an den Kunststoffpackmitteln führen kann. Eine Sterilfiltration von wässrigen Hyaluronsäurelösungen hingegen ist auf Grund der recht hohen Viskosität der Lösungen praktisch nicht oder nur sehr aufwendig möglich. Bei der Sterilfiltration werden nur mikrobielle Lebensformen ab einer bestimmten Größe entfernt. Viren können durch Sterilfiltration nicht entfernt oder inaktiviert werden.

Neben diesen physikalischen Sterilisationsverfahren werden oft auch chemische Verbindungen zur Sterilisation von Medizinprodukten eingesetzt. Dazu gehören zum Beispiel Formaldehyd, Glutardialdehyd, o-Phthaldialdehyd. Die Sterilisation mit Aldehyden ist mit dem Nachteil verbunden, dass diese nach der Sterilisation wieder entfernt werden müssen, um bei der Anwendung am Menschen Schädigungen zu vermeiden. Bei endverpackten wässrigen Hyaluronsäurelösungen ist dadurch eine Sterilisation mit Aldehyden ausgeschlossen, da diese aus endverpackten Hyaluronsäurelösungen nicht wieder entfernt werden können.

Sehr wirksame Sterilisationsmittel stellen oxidierende Agenzien, wie Wasserstoffperoxid, Perameisensäure, Peressigsäure, Hypochlorid und Hypochlorid abspaltende Substanzen, wie Chloramin T2 oder Trichlorisocyanursäure, dar. Nachteilig an diesen Agenzien ist, dass diese einen erheblichen oxidativen Abbau der gelösten Hyaluronsäure verursachen. Weiterhin können nicht umgesetzte Reste der oxidierenden Agenzien in der endverpackten Hyaluronsäurelösung verbleiben, die gegebenenfalls lokaltoxisch sein können.

Aus der pharmazeutischen Industrie ist bekannt, dass wässrige Proteinlösungen, wie zum Beispiel Impfseren, gegenüber oxidierenden Sterilisationsmitteln und verschiedenen physikalischen Sterilisationsverfahren, zum Beispiel der Sterilisation mit Gammastrahlung, sehr empfindlich sind. Aus diesem Grund werden diese wässrigen Proteinlösungen zuerst sterilfiltriert und dann zur Inaktivierung von Viren mit geringen Mengen an β-Propiolacton versetzt. β-Propiolacton acyliert die Aminogruppen der DNA/RNA oder von Proteinen der Viren. Das als Lösungsmittel enthaltene Wasser ist in der Lage, das β-Propiolacton langsam zu zersetzen, so dass in den wässrigen Proteinlösungen nach kurzer Zeit kein aktives β-Propiolacton mehr enthalten ist. Bisher ist bekannt, dass gasförmiges β-Propiolacton Endoporen irreversibel inaktivieren kann (R. K. Hoffmann, B. Warshowsky: Beta-Propiolactone Vapor as a Disinfectant. Appl. Microbiol. 1958 Sept.; 6(5): 358-362). Weiterhin ist bekannt, dass β-Propiolacton Endoporen in nicht wässrigen organischen Monomeren/Monomergemischen und pastenförmigen Zementen, die organische Monomere enthalten, inaktiviert (EP 2 596 812 B1).

Neben den vegetativen Formen der Mikroorganismen gibt es jedoch auch generative Formen, wie zum Beispiel Endosporen. Sie stellen generative Überdauerungsformen von Mikroorganismen dar und werden von Gram-positiven Bakterien, besonders der Gattungen Bacillus und Clostridium, gebildet, um ungünstige Lebensbedingungen überdauern zu können. Endosporen haben im Ruhezustand keinen aktiven Stoffwechsel und besitzen eine mehrschichtige Sporenhülle, die den Sporen-Kern vor Einwirkung von Chemikalien und anderen Umwelteinflüssen weitgehend schützt. Dadurch sind Endosporen extrem widerstandsfähig gegenüber der Einwirkung von Hitze und von Chemikalien (Borick, P. M.: Chemical sterilizers. Adv. Appl. Microbiol. 10 (1968) 291-312 ; Gould, G. W.: Recent advances in the understanding of resistance and dormacy in bacterial spores. J. Appl. Bacteriol. 42 (1977) 297-309 ; Gould, G. W.: Mechanisms of resistance and dormancy. p. 173-209. In Hurst, A. and Gould, G. W. (ed.), The bacterial spore. vol. 2 Academic Press, Inc. New York, 1983). Endosporen werden aufgrund ihrer hohen Widerstandsfähigkeit als Bioindikatoren für die Validierung und die Wirksamkeitskontrolle von Sterilisationsprozessen eingesetzt. Es wird dabei davon ausgegangen, dass eine Inaktivierung der Endosporen eine Abtötung aller vegetativen mikrobiellen Lebensformen abbildet. Endosporen von Gram-positiven Bakterien gehören der internationalen Resistenzstufe III an. Zu den Resistenzstufen I gehören nichtsporenbildende Bakterien und vegetative Formen von Sporenbildnern und zur Resistenzstufe II zählen Sporen, die in strömenden Wasserdampf bei 105 °C innerhalb weniger Minuten abgetötet werden. Bei einer Sterilisation müssen nach DAB (Deutsches Arzneimittelbuch) 2008 alle Mikroorganismen der Resistenzstufen I-III abgetötet beziehungsweise irreversibel inaktiviert werden.

Erfindungsgemäß ist weiterhin ein Verfahren zur Sterilisation der erfindungsgemäßen Polymerlösung für eine Viscosupplementation. Dieses Verfahren ist dadurch charakterisiert, dass ein Gemisch hergestellt wird, das mindestens ein zu mindestens teilweise in Wasser lösliches Polysaccharid, das aus der Gruppe ausgewählt ist, die aus Alkalisalzen der Hyaluronsäure, Alkalisalzen der Carboxymethylcellulose, Methycellulose und Hydroxyethylcellulose besteht, ein in Wasser lösliches Alkalisalz oder Erdalkalisalz der Polystyrensulfonsäure und Wasser mit mindestens 0,5 Gew.-% β-Propiolacton enthält, und die Polymerlösung mindestens 24 Stunden bei 4-40 °C gelagert wird. Überraschend wurde gefunden, dass die Sterilisation der erfindungsgemäßen Polymerlösung mit β-Propiolacton gelingt, ohne dass unerwünschte Verfärbungen auftreten. Weiterhin wurde überraschend gefunden, dass bei der Sterilisation mit β-Propiolacton Polymerlösungen, die Hyaluronsäure, Carboxymethylcellulose, Methylcellulose, Hydroxyethylcellulse in Kombination mit Polystyrolsulfonsäure enthalten, nicht ausfallen, sondern sichtklar gelöst bleiben.

Bevorzugt werden 0,5-2,0 Gew.-% β-Propiolacton zur Sterilisation der wässrigen Polymerlösung zugesetzt. In diesem Konzentrationsbereich ist eine sichere Inaktivierung von Endosporen gewährleistet.

Die erfindungsgemäße Polymerlösung wird als Mittel zur Viscosupplementation und als pharmazeutischer Wirkstoffträger bereitgestellt.

Die Erfindung wird durch nachstehende Beispiele erläutert, ohne dass diese jedoch die Erfindung beschränken.

Es wurden folgende Polysaccharide bzw. Polysacchariderivate für die nachfolgend beschriebenen Versuche eingesetzt:
- NaHya:: Natriumsalz der Hyaluronsäure (Mₙ ∼ 0,9 Millionen Dalton),
- CMC:: Natriumsalz der Carboxymethylcellulose (Mₙ ∼ 90.000 Dalton),
- MC:: Methylcellulose (SM-4000),
- HEC:: Hydroxyethylcellulose (60SH-4000)
- PSS1:: Natriumsalz der Polystyrensulfonsäure (Mₙ∼ 70.000 Dalton)
- PSS2:: Natriumsalz der Polystyrensulfonsäure (Mₙ∼ 1.300.000 Dalton)

Es wurde ein Phosphat-Puffer mit einem pH-Wert von 7,4 hergestellt. Dazu wurden 1,65 g Kaliumhydrogenphosphat und 9,71 g Dinatriumhydrogenphosphat-Dihydrat in einem Liter destillierten Wasser gelöst.

### Beispiel 1

Es wurden in Rollrandgläsern jeweils 5,0 ml des Phosphatpuffers mit einem pH-Wert von 7,4 vorgelegt. In diesen Pufferlösungen wurden jeweils 0,25 Gew.-%, 0,5 Gew.-%, 1,0 Gew.-% und 2,0 Gew.-% Polysaccharid zusammen mit dem Natriumsalz der Polystyrolsulfonsäure (PSS1 und PSS2) bei Raumtemperatur gelöst. Nach 24 Stunden wurden die Polymerlösungen visuell begutachtet.

| Polymerlösung | Zusammensetzung der Polymerlösung | | Aussehen |
|---|---|---|---|
| 1 | 0,25 Gew.-% NaHya | 0,25 Gew.-% PSS1 | sichtklar |
| 2 | 0,25 Gew.-% CMC | 0,25 Gew.-% PSS1 | sichtklar |
| 3 | 0,25 Gew.-% MC | 0,25 Gew.-% PSS1 | sichtklar |
| 4 | 0,25 Gew.-% HEC | 0,25 Gew.-% PSS1 | sichtklar |

| Polymerlösung | Zusammensetzung der Polymerlösung | | Aussehen |
|---|---|---|---|
| 5 | 0,25 Gew.-% NaHya | 0,25 Gew.-% PSS2 | sichtklar |
| 6 | 0,25 Gew.-% CMC | 0,25 Gew.-% PSS2 | sichtklar |
| 7 | 0,25 Gew.-% MC | 0,25 Gew.-% PSS2 | sichtklar |
| 8 | 0,25 Gew.-% HEC | 0,25 Gew.-% PSS2 | sichtklar |

| Polymerlösung | Zusammensetzung der Polymerlösung | | Aussehen |
|---|---|---|---|
| 9 | 0,5 Gew.-% NaHya | 0,5 Gew.-% PSS1 | sichtklar |
| 10 | 0,5 Gew.-% CMC | 0,5 Gew.-% PSS1 | sichtklar |
| 11 | 0,5 Gew.-% MC | 0,5 Gew.-% PSS1 | sichtklar |
| 12 | 0,5 Gew.-% HEC | 0,5 Gew.-% PSS1 | sichtklar |

| Polymerlösung | Zusammensetzung der Polymerlösung | | Aussehen |
|---|---|---|---|
| 13 | 0,5 Gew.-% NaHya | 0,5 Gew.-% PSS2 | sichtklar |
| 14 | 0,5 Gew.-% CMC | 0,5 Gew.-% PSS2 | sichtklar |
| 15 | 0,5 Gew.-% MC | 0,5 Gew.-% PSS2 | sichtklar |
| 16 | 0,5 Gew.-% HEC | 0,5 Gew.-% PSS2 | sichtklar |

| Polymerlösung | Zusammensetzung der Polymerlösung | | Aussehen |
|---|---|---|---|
| 17 | 1,0 Gew.-% NaHya | 1,0 Gew.-% PSS1 | sichtklar |
| 18 | 1,0 Gew.-% CMC | 1,0 Gew.-% PSS1 | sichtklar |
| 19 | 1,0 Gew.-% MC | 1,0 Gew.-% PSS1 | sichtklar |
| 20 | 1,0 Gew.-% HEC | 1,0 Gew.-% PSS1 | sichtklar |

| Polymerlösung | Zusammensetzung der Polymerlösung | | Aussehen |
|---|---|---|---|
| 21 | 1,0 Gew.-% NaHya | 1,0 Gew.-% PSS2 | sichtklar |
| 22 | 1,0 Gew.-% CMC | 1,0 Gew.-% PSS2 | sichtklar |
| 23 | 1,0 Gew.-% MC | 1,0 Gew.-% PSS2 | sichtklar |
| 24 | 1,0 Gew.-% HEC | 1,0 Gew.-% PSS2 | sichtklar |

### Beispiel 2

Es wurde Polymerlösungen analog dem Beispiel 1 hergestellt, die jedoch zusätzlich das Antibiotikum Doxycyclin enthielten. Dazu wurde Doxycyclin-Hyalate (Doxycyclinhydrochlorid-Hemiethanolat-Hemihydrat) verwendet. Es wurden die Polymerlösungen nach 24 Stunden Lagerung bei Raumtemperatur visuell geprüft.

| Polymerlösung | Zusammensetzung der Polymerlösung | | | Aussehen |
|---|---|---|---|---|
| 25 | 0,5 Gew.-% NaHya | 0,5 Gew.-% PSS1 | 0,01 Gew.% Doxycyclin-Hyalat | sichtklar |
| 26 | 0,5 Gew.-% CMC | 0,5 Gew.-% PSS1 | 0,01 Gew.% Doxycyclin-Hyalat | sichtklar |
| 27 | 0,5 Gew.-% MC | 0,5 Gew.-% PSS1 | 0,01 Gew.% Doxycyclin-Hyalat | sichtklar |
| 28 | 0,5 Gew.-% HEC | 0,5 Gew.-% PSS1 | 0,01 Gew.% Doxycyclin-Hyalat | sichtklar |

### Beispiel 3

Es wurde Polymerlösungen analog dem Beispiel 1 hergestellt, die jedoch zusätzlich das Antibiotikum Gentamicinsulfat enthielten. Dazu wurde Gentamicinsulfat (Akitivitätskoeffizient 580) verwendet. Es wurden die Polymerlösungen nach 24 Stunden Lagerung bei Raumtemperatur visuell geprüft.

| Polymerlösung | Zusammensetzung der Polymerlösung | | | Aussehen |
|---|---|---|---|---|
| 25 | 0,5 Gew.-% NaHya | 0,5 Gew.-% PSS1 | 0,01 Gew.-% Gentamicinsulfat- | sichtklar |
| 26 | 0,5 Gew.-% CMC | 0,5 Gew.-% PSS1 | 0,01 Gew.-% Gentamicinsulfat- | sichtklar |
| 27 | 0,5 Gew.-% MC | 0,5 Gew.-% PSS1 | 0,01 Gew.-% Gentamicinsulfat- | sichtklar |
| 28 | 0,5 Gew.-% HEC | 0,5 Gew.-% PSS1 | 0,01 Gew.-% Gentamicinsulfat- | sichtklar |

### Beispiel 4

Es wurde Polymerlösungen analog dem Beispiel 1 hergestellt, die jedoch zusätzlich das Antiphlogistikum Dexamethasonphosphat enthielten. Dazu wurde das Natriumsalz des Dexamethasonphosphats (NaDexP) verwendet. Es wurden die Polymerlösungen nach 24 Stunden Lagerung bei Raumtemperatur visuell geprüft.

| Polymerlösung | Zusammensetzung der Polymerlösung | | | Aussehen |
|---|---|---|---|---|
| 25 | 0,5 Gew.-% NaHya | 0,5 Gew.-% PSS1 | 0,01 Gew.% NaDexP | sichtklar |
| 26 | 0,5 Gew.-% CMC | 0,5 Gew.-% PSS1 | 0,01 Gew.% NaDexP | sichtklar |
| 27 | 0,5 Gew.-% MC | 0,5 Gew.-% PSS1 | 0,01 Gew.% NaDexP | sichtklar |
| 28 | 0,5 Gew.-% HEC | 0,5 Gew.-% PSS1 | 0,01 Gew.% NaDexP | sichtklar |

### Beispiel 5

Es wurde eine 0,25 Gew.-%ige Lösung von NaHya mit Phosphat-Puffer pH 7,4 hergestellt. Zu jeweils 40,0 g der NaHya-lösung wurde 10 mg, 1,0 mg, 0,1 mg und 0,01 mg PSS1 gegeben. Für den Zusatz von 0,1 mg und 0,01 mg PSS1 wurde eine Lösung von 10 mg PSS1 in Phosphatpuffer hergestellt und entsprechende Aliquote dieser Lösung der NaHya-Lösung zugesetzt. Es wurde eine Lösung von 26,7 IU/µl einer bovinen Hyaluronidase (Fa. Kraeber, 329 IU/mg) in Phosphat-Puffer pH 7,4 angesetzt. Es wurden dann jeweils 150 µl dieser Hyaluronidaselösung zu 40 g der NaHya-PSS1-Lösungen zugesetzt. Die Lösungen wurden dann für 15 Minuten auf 37 °C in einem Ubbelohde-Viskosimeter (Kapillare I) temperiert. Dann erfolgte die Bestimmung der Durchlaufzeit der Polymerlösung. Die Polymerlösungen wurden dann im Ubbelohde-Viskosimeter bei 37 °C belassen. Die Durchlaufzeit der Polymerlösung wurde nach jeder weiteren Stunde gemessen. Zusätzlich wurde als Referenz die Durchlaufzeit einer 0,25 Gew.-%igen NaHya-lösung mit und Zusatz von Hyaluronidase gemessen.

| | Zusammensetzung der 0,25 Gew.-%igen NaHY-Polymerlösungen | | | | | |
|---|---|---|---|---|---|---|
| NaHya | 0,25 Gew.-% | 0,25 Gew.-% | 0,25 Gew.-% | 0,25 Gew.-% | 0,25 Gew.-% | 0,25 Gew.-% |
| Hyaluronidase | - | + | + | + | + | + |
| PSS1 | - | - | 0,025 Gew.-% | 0,0025 Gew.-% | 0,00025 Gew.-% | 0,000025 Gew.-% |

| Zeit [h] | Durchlaufzeit [min] | | | | | |
|---|---|---|---|---|---|---|
| 0,25 | 19 min 12 s | 12 min 28 s | 22 min 5 s | 22 min 20 s | 18 min 56 s | 9 min 14 s |
| 1,0 | 19 min 26 s | 7 min 16 s | 22 min 0 s | 22 min 5 s | 19 min 1 s | 6 min 15 s |
| 2,0 | 19 min 17 s | 5 min 26 s | 21 min 50 s | 21 min 58 s | 19 min 12 s | 4 min 52 s |
| 3,0 | 19 min 19 s | 4 min 31 | 21 min 42 s | 22 min 3 s | 19 min 13 s | 3 min 56 |
| 4,0 | 19 min 11 s | 3 min 55 s | 21 min 40 s | 22 min 6 s | 19 min 13 s | 3 min 31 s |
| 5,0 | 19 min 10 s | 3 min 31 s | 21 min 44 s | 22 min 11 s | 19 min 22 s | 3 min 10 s |

Die Durchlaufzeiten der Polymerlösungen sind proportional zur Molmasse der gelösten Polymere. Eine Abnahme der Durchlaufzeit ist auf eine Verringerung der Molmassen zurück zu führen. Die Ergebnisse zeigen, dass Polymerlösungen, die Hyaluronsäure und Polystyrensulfonsäure enthalten, im Untersuchungszeitraum von 5 Stunden praktisch keine Verringerung der Durchlaufzeiten im Ubbelohde-Viskosimeter aufwiesen. Die in der Polymerlösung enthaltene Hyaluronsäure wird offensichtlich durch die Hyaluronidase nicht degradiert.

Zur Kontrolle wurde in einem weiteren Versuch der Einfluss der Hyaluronidase auf die Polystyrensulfonsäure untersucht. Der Versuchsaufbau entsprach dem vorangegangenen Versuch.

| | Zusammensetzung der Polymerlösungen | |
|---|---|---|
| PSS1 | 0,25 Gew.% | - |
| PSS2 | - | 0,25 Gew.% |

| Zeit [h] | Durchlaufzeit [min] | |
|---|---|---|
| 0,25 | 1 min 36 s | 4 min 28 s |
| 1 | 1 min 37 s | 4 min 27 s |
| 2 | 1 min 37 s | 4 min 29 s |
| 3 | 1 min 36 s | 4 min 26 s |
| 4 | 1 min 37 s | 4 min 28 s |
| 5 | 1 min 37 s | 4 min 26 s |

Die Ergebnisse zeigen, dass wässrige Polystyrolsulfonsäure-Lösungen bei 37 °C nicht durch Hyaluronidase degradiert werden können.

### Beispiel 6

Es wurde eine 0,25 Gew.%ige Lösung von NaHya mit Phosphat-Puffer pH 7,4 hergestellt. Zu jeweils 40,0 g der NaHya-lösung wurde 10 mg, 1,0 mg, 0,1 mg und 0,01 mg PSS2 gegeben. Für den Zusatz von 0,1 mg und 0,01 mg PSS1 wurde eine Lösung von 10 mg PSS2 in Phosphatpuffer hergestellt und entsprechende Aliquote dieser Lösung der NaHya-Lösung zugesetzt. Es wurde eine Lösung von 26,7 IU/µl einer bovinen Hyaluronidase (Fa. Kraeber, 329 IU/mg) in Phosphat-Puffer pH 7,4 angesetzt. Es wurden dann jeweils 150 µl dieser Hyaluronidaslösung zu 40 g der NaHya-PSS2-Lösungen zugesetzt. Die Lösungen wurden dann für 15 Minuten auf 37 °C in einem Ubbelohde-Viskosimeter (Kapillare I) temperiert. Dann erfolgte die Bestimmung der Durchlaufzeit der Polymerlösung. Die Polymerlösungen wurden dazu im Ubbelohde-Viskosimeter bei 37 °C belassen. Die Durchlaufzeit der Polymerlösung wurde nach jeder weiteren Stunde gemessen. Zusätzlich wurde als Referenz die Durchlaufzeit einer 0,25 Gew.-%igen NaHya-lösung mit und Zusatz von Hyaluronidase gemessen.

| | Zusammensetzung der 0,25 Gew.-%igen NaHY-Polymerlösungen | | | | | |
|---|---|---|---|---|---|---|
| NaHya | 0,25 Gew.-% | 0,25 Gew.-% | 0,25 Gew.-% | 0,25 Gew.-% | 0,25 Gew.-% | 0,25 Gew.-% |
| Hyaluronidase | - | + | + | + | + | + |
| PSS2 | - | - | 0,025 Gew.-% | 0,0025 Gew.-% | 0,00025 Gew.-% | 0,000025 Gew.-% |

| Zeit [h] | Durchlaufzeit [min] | | | | | |
|---|---|---|---|---|---|---|
| 0,25 | 19 min 12 s | 12 min 28 s | 19 min 52 s | 22 min 26 s | 22 min 12 s | 18 min 17 s |
| 1,0 | 19 min 26 s | 7 min 16 s | 20 min 2 s | 22 min 16 s | 22 min 5 s | 18 min 05 s |
| 2,0 | 19 min 17 s | 5 min 26 s | 19 min 50 s | 22 min 24 s | 22 min 3 s | 18 min 10 s |
| 3,0 | 19 min 19 s | 4 min 31 | 19 min 57 s | 22 min 24 s | 22 min 5 s | 17 min 58 s |
| 4,0 | 19 min 11 s | 3 min 55 s | 19 min 53 s | 22 min 22 s | 22 min 3 s | 18 min 5 s |
| 5,0 | 19 min 10 s | 3 min 31 s | 20 min 1 s | 22 min 23 s | 22 min 4 s | 18 min 7 s |

Die Durchlaufzeiten der Polymerlösungen sind proportional zur Molmasse der gelösten Polymere. Eine Abnahme der Durchlaufzeit ist auf eine Verringerung der Molmassen zurück zu führen. Die Ergebnisse zeigen, dass Polymerlösungen, die Hyaluronsäure und Polystyrensulfonsäure enthalten im Untersuchungszeitraum von 5 Stunden praktisch keine Verringerung der Durchlaufzeiten im Ubbelohde-Viskosimeter aufwiesen. Die Hyaluronsäure wird in den Hyaluronsäure/Polystyrolsulfonsäure-Lösungen offensichtlich nicht durch die Hyaluronidase degradiert.

### Beispiel 7

Es wurden zuerst mit jeweils 30,0 ml Phosphat-Puffer (pH-Wert von 7,4) Lösungen der Polysaccharide mit Polystyrensulfonsäure hergestellt. Zu jeweils 5,0 ml der Polysaccharidlösungen in einem sterilen 25 ml Falcon-Plastiktube wurden 10⁶ KBE einer Sporensuspension von *Bacillus atropheus* gegeben. Danach wurden die Sporen mit Hilfe eines Vortex-Mischgerätes homogen suspendiert. Anschließend wurden 0,5 Gew.-%, 1,0 Gew.-% und 2,0 % β-Propiolacton zu jeweils 5,0 ml der zuvor mit Sporen vermischten Polysaccharid-Lösung zugesetzt. Danach wurde nochmals mit einem Vortex-Mischgerät homogenisiert. Als Positiv-Kontrolle wurden nicht mit β-Propiolacton behandelte Polysaccharidlösungen mitgeführt. Nach 48 Stunden Lagerung der Polysaccharidlösungen bei Raumtemperatur erfolgte eine Prüfung auf Sterilität gemäß DIN EN ISP 11737, Teil 2. Es wurden dazu Doppelbestimmungen ausgeführt.

| Zusammensetzung der Polymerlösung | | Ergebnisse der Sterilprüfung | | | |
|---|---|---|---|---|---|
| | | Konzentration β-Propiolacton [Gew.-%] | | | |
| | | 0,0 (Positiv-Kontrolle) | 0,5 | 1,0 | 2,0 |
| 0,5 Gew.-% NaHya | 0,5 Gew.-% PSS1 | +/+ | -/- | -/- | -/- |
| 0,5 Gew.-% CMC | 0,5 Gew.-% PSS1 | +/+ | -/- | -/- | -/- |
| 0,5 Gew.-% MC | 0,5 Gew.-% PSS1 | +/+ | -/- | -/- | -/- |
| 0,5 Gew.-% HEC | 0,5 Gew.-% PSS1 | +/+ | -/- | -/- | -/- |
| 0,5 Gew.-% NaHya | 0,5 Gew.-% PSS2 | +/+ | -/- | -/- | -/- |
| 0,5 Gew.-% CMC | 0,5 Gew.-% PSS2 | +/+ | -/- | -/- | -/- |
| 0,5 Gew.-% MC | 0,5 Gew.-% PSS2 | +/+ | -/- | -/- | -/- |
| 0,5 Gew.-% HEC | 0,5 Gew.-% PSS2 | +/+ | -/- | -/- | -/- |

| | | | | | |
|---|---|---|---|---|---|
| (+) Wachstum (-) kein Wachstum | | | | | |

Die mit β-Propiolacton sterilisierten Polysaccharidlösungen zeigten keinerlei Verfärbung gegenüber den als Positiv-Kontrolle mitgeführten unbehandelten Polysaccharidlösungen.

## Patentansprüche

1. Polymerlösung zur Verwendung bei der therapeutischen Viscosupplementation enthaltend
mindestens ein zu mindestens teilweise in Wasser lösliches Polysaccharid, das aus der Gruppe ausgewählt ist, die aus Alkalisalzen der Hyaluronsäure, Alkalisalzen der Carboxymethylcellulose, Methylcellulose und Hydroxyethylcellulose besteht und Wasser,
**dadurch gekennzeichnet, dass**
sie ferner mindestens ein in Wasser lösliches Alkalisalz oder Erdalkalisalz der Polystyrensulfonsäure enthält und
die Lösung sichtklar ist.

2. Polymerlösung zur Verwendung bei der therapeutischen Viscosupplementation nach Anspruch 1 zur Verwendung bei der Behandlung von Arthrose.

3. Polymerlösung zur Verwendung bei der therapeutischen Viscosupplementation nach Anspruch 1 oder 2 enthaltend
3-Hydroxypropionsäure.

4. Polymerlösung zur Verwendung bei der therapeutischen Viscosupplementation nach einem der vorhergehenden Ansprüche, **dadurch gekennzeichnet, dass** das Massenverhältnis von Polysaccharid oder einer Mischung aus mindestens zwei Polysacchariden zu dem Alkalisalz oder Erdalkalisalz der Polystyrensulfonsäure in einem Bereich von 1 zu 1 bis 1 zu 0,01 liegt.

5. Polymerlösung zur Verwendung bei der therapeutischen Viscosupplementation nach einem der vorhergehenden Ansprüche, **dadurch gekennzeichnet, dass** der Gesamtpolymergehalt in der Polymerlösung von 0,1 bis 10 Gew.-% beträgt.

6. Polymerlösung zur Verwendung bei der therapeutischen Viscosupplementation nach einem der vorhergehenden Ansprüche, **dadurch gekennzeichnet, dass** die Polymerlösung ferner Antiphlogistika, Antibiotika, Immunsuppressiva und Cytostatika enthält.

7. Polymerlösung zur Verwendung bei der therapeutischen Viscosupplementation nach Anspruch 6, **dadurch gekennzeichnet, dass**
die Polymerlösung Dexamethasonphosphat, Triamcinolonphosphat, Doxycyclin, Chlortetracyclin, Oxytetracyclin, Doxorubicin, Ciclosporin, Methotrexat, Leflunomid, Azathioprin, Mitomycin C, Tacrolimus, Sirolimus und Everolimus enthält.

8. Polymerlösung zur Verwendung bei der therapeutischen Viscosupplementation nach einem der vorhergehenden Ansprüche, bestehend aus
a) mindestens einem zu mindestens teilweise in Wasser löslichen Polysaccharid,
b) mindestens einem in Wasser lösliches Alkalisalz oder Erdalkalisalz der Polystyrensulfonsäure,
c) Wasser,
d) optional 3- Hydroxypropionsäure,
e) optional mindestens einem Antiphlogistikum,
f) optional mindestens einem Antibiotikum,
g) optional mindestens einem Immunsuppressivum,
h) optional mindestens einem Cytostatikum.

9. Verfahren zur Herstellung einer sterilen, wässrigen Polymerlösung für eine Viscosupplementation enthaltend mindestens ein zu mindestens teilweise in Wasser lösliches Polysaccharid, das aus der Gruppe ausgewählt ist, die aus Alkalisalzen der Hyaluronsäure, Alkalisalzen der Carboxymethylcellulose, Methycellulose und Hydroxyethylcellulose besteht, Wasser, mindestens ein in Wasser lösliches Alkalisalz oder Erdalkalisalz der Polystyrensulfonsäure und 3-Hydroxypropionsäure und wobei die Lösung sichtklar ist, **dadurch gekennzeichnet, dass**
a) mindestens ein zu mindestens teilweise in Wasser lösliches Polysaccharid, das aus der Gruppe ausgewählt ist, die aus Alkalisalzen der Hyaluronsäure, Alkalisalzen der Carboxymethylcellulose, Methycellulose und Hydroxyethylcellulose besteht,
b) ein in Wasser lösliches Alkalisalz oder Erdalkalisalz der Polystyrensulfonsäure und
c) Wasser mit mindestens 0,5 Gew.-% β-Propiolacton
gemischt werden und
die so entstandene Polymerlösung mindestens 24 Stunden bei 4-40 °C gelagert wird.

10. Verfahren nach Anspruch 9 **dadurch gekennzeichnet, dass** 0,5-2,0 Gew.-% β-Propiolacton zur Sterilisation zugesetzt wird.

## Claims

1. A polymer solution for use in the therapeutic viscosupplementation, containing at least one at least partially water-soluble polysaccharide, which is selected from the group consisting of alkali salts of the hyaluronic acid, alkali salts of the carboxymethyl cellulose, methylcellulose, and hydroxyethyl cellulose, and water,
**characterised in that**
it further contains at least one water-soluble alkali salt or alkaline earth salt of the polystyrene sulfonic acid, and
the solution is visually clear.

2. The polymer solution for use in the therapeutic viscosupplementation according to claim 1 for use in the treatment of arthritis.

3. The polymer solution for use in the therapeutic viscosupplementation according to claim 1 or 2, containing
3-hydroxypropionic acid.

4. The polymer solution for use in the therapeutic viscosupplementation according to any one of the preceding claims, **characterised in that** the mass ratio of polysaccharide or of a mixture of at least two polysaccharides and the alkali salt or alkaline earth salt of the polystyrene sulfonic acid lies in a range of 1 to 1 to 1 to 0.01.

5. The polymer solution for use in the therapeutic viscosupplementation according to any one of the preceding claims, **characterised in that** the total polymer content in the polymer solution is 0.1 to 10% by weight.

6. The polymer solution for use in the therapeutic viscosupplementation according to any one of the preceding claims, **characterised in that** the polymer solution further contains antiphlogistic agents, antibiotics, immunosuppressants, and cytostatic agents.

7. The polymer solution for use in the therapeutic viscosupplementation according to claim 6, **characterised in that**
the polymer solution contains dexamethasone phosphate, triamcinolone phosphate, doxycycline, chlortetracycline, oxytetracycline, doxorubicin, cyclosporine, methotrexate, leflunomide, azathioprine, mitomycin C, tacrolimus, sirolimus, and everolimus.

8. The polymer solution for use in the therapeutic viscosupplementation according to any one of the preceding claims, consisting of
a) at least one at least partially water-soluble polysaccharide,
b) at least one water-soluble alkali salt or alkaline earth salt of the polystyrene sulfonic acid,
c) water,
d) optionally 3-hydroxypropionic acid,
e) optionally at least one antiphlogistic agent,
f) optionally at least one antibiotic,
g) optionally at least one immunosuppressant,
h) optionally at least one cytostatic agent.

9. A method for producing a sterile, aqueous polymer solution for a viscosupplementation containing at least one at least partially water-soluble polysaccharide, which is selected from the group consisting of alkali salts of the hyaluronic acid, alkali salts of the carboxymethyl cellulose, methylcellulose, and hydroxyethyl cellulose, water, at least one water-soluble alkali salt or alkaline earth salt of the polystyrene sulfonic acid, and 3-hydroxypropionic acid, and wherein the solution is visually clear, **characterised in that**
a) at least one at least partially water-soluble polysaccharide selected from the group consisting of alkali salts of the hyaluronic acid, alkali salts of the carboxymethyl cellulose, methylcellulose, and hydroxyethyl cellulose,
b) a water-soluble alkali salt or alkaline earth salt of the polystyrene sulfonic acid, and
c) water with at least 0.5% by weight of β-propiolactone are mixed, and
the polymer solution created in this way is stored for at least 24 hours at 4-40°C.

10. The method according to claim 9, **characterised in that** 0.5-2.0% by weight of β-propiolactone are added for the sterilisation.

## Revendications

1. Solution polymère destinée à être utilisée dans le traitement visco-supplétif, contenant
au moins un polysaccharide au moins partiellement soluble dans l'eau, lequel est sélectionné dans le groupe composé de sels alcalins de l'acide hyaluronique, de sels alcalins de la carboxyméthylcellulose, la méthylcellulose et l'hydroxyéthylcellulose, et
de l'eau,
**caractérisée en ce que**
la solution contient en outre au moins un sel alcalin ou sel alcalino-terreux de l'acide sulfonique de polystyrène, soluble dans l'eau, et
la solution est transparente.

2. Solution polymère destinée à être utilisée dans le traitement visco-supplétif conformément à la revendication n°1 pour un usage dans le traitement de l'arthrose.

3. Solution polymère destinée à être utilisée dans le traitement visco-supplétif conformément à la revendication n°1 ou n°2 contenant de l'acide 3-hydroxypropanoïque.

4. Solution polymère destinée à être utilisée dans le traitement visco-supplétif conformément à l'une des revendications précédentes, **caractérisée en ce que** le rapport de masse du polysaccharide ou d'un mélange d'au moins deux polysaccharides par rapport au sel alcalin ou sel alcalino-terreux de l'acide sulfonique de polystyrène est de l'ordre de 1:1 à 1:0,01.

5. Solution polymère destinée à être utilisée dans le traitement visco-supplétif conformément à l'une des revendications précédentes, **caractérisée en ce que** la teneur totale en polymère dans la solution polymère est de 0,1 à 10 % en poids.

6. Solution polymère destinée à être utilisée dans le traitement visco-supplétif conformément à l'une des revendications précédentes, **caractérisée en ce que** la solution polymère contient en outre des anti-inflammatoires, des antibiotiques, des immunosuppresseurs et des cytostatiques.

7. Solution polymère destinée à être utilisée dans le traitement visco-supplétif conformément à la revendication n°6, **caractérisée en ce que** la solution polymère contient du phosphate de dexaméthasone, du phosphate de triamcinolone, de la doxycycline, de la chlortétracycline, de l'oxytétracycline, de la doxorubicine, de la ciclosporine, du méthotrexate, du léflunomide, de l'azathioprine, de la mitomycine C, du tacrolimus, du sirolimus et de l'éverolimus.

8. Solution polymère destinée à être utilisée dans le traitement visco-supplétif conformément à l'une des revendications précédentes, composée
a) au moins d'un polysaccharide au moins partiellement soluble dans l'eau,
b) au moins d'un sel alcalin ou sel alcalino-terreux de l'acide sulfonique de polystyrène, soluble dans l'eau,
c) d'eau,
d) facultativement d'acide 3-hydroxypropanoïque,
e) facultativement au moins d'un anti-inflammatoire,
f) facultativement au moins d'un antibiotique,
g) facultativement au moins d'un immunosupresseur,
h) facultativement au moins d'un cytostatique.

9. Procédé de fabrication d'une solution polymère aqueuse, stérile pour un traitement visco-supplétif, contenant au moins un polysaccharide au moins partiellement soluble dans l'eau, lequel est sélectionné dans le groupe composé de sels alcalins de l'acide hyaluronique, de sels alcalins de la carboxyméthylcellulose, la méthylcellulose et l'hydroxyéthylcellulose, de l'eau, au moins un sel alcalin ou sel alcalino-terreux de l'acide sulfonique de polystyrène et de l'acide 3-hydroxypropanoïque, soluble dans l'eau, la solution étant transparente, **caractérisé en ce que**
a) au moins un polysaccharide au moins partiellement soluble dans l'eau, lequel est sélectionné dans le groupe composé de sels alcalins de l'acide hyaluronique, de sels alcalins de la carboxyméthylcellulose, la méthylcellulose et l'hydroxyéthylcellulose,
b) un sel alcalin ou sel alcalino-terreux de l'acide sulfonique de polystyrène, soluble dans l'eau,
et
c) de l'eau avec au moins 0,5% en poids de bêta-propiolactone sont mélangés et
la solution polymère ainsi produite est stockée à une température de 4 à 40°C pendant au moins 24 heures.

10. Procédé conformément à la revendication n°9, **caractérisé en ce que** 0,5 à 2,0% en poids de bêta-propiolactone sont ajoutés pour la stérilisation.
